# FASCICULE DE BREVET EUROPEEN

(11) **EP 3 097 073 B1**
(45) Date de publication et mention de la délivrance du brevet: **25.12.2019**
(21) Numéro de dépôt: 15704468.6
(22) Date de dépôt: 20.01.2015
(51) Int. Cl.: C07C 51/56, C07C 51/573, C07C 57/04

(54) **SYNTHÈSE D'ANHYDRIDE (MÉTH)ACRYLIQUE PAR TRANSANHYDRIFICATION**
SYNTHESE VON (METH)ACRYLSÄUREANHYDRID DURCH TRANSANHYDRISIERUNG
SYNTHESIS OF (METH)ACRYLIC ANHYDRIDE BY TRANSANHYDRIZATION

(30) Priorité: 20.01.2014 FR 1400106
(43) Date de publication de la demande: 30.11.2016
(73) Titulaire: Rhodia Operations, 75009 Paris (FR)
(72) Inventeur: ARDAUD, Pierre, F-69110 Sainte-Foy-les-Lyon (FR); VIDAL, Thierry, F-69003 Lyon (FR); RACHED, Rabih, 201702 Shanghai (FR)
(74) Mandataire: Cordier, Pascal Christian
(86) Numéro de dépôt international: PCT/EP2015/050972
(87) Numéro de publication internationale: WO 2015/107210

(56) Documents cités:
- EP-A1- 0 196 520
- WO-A1-2009/098422
- GB-A- 538 310
- US-A1- 2002 161 260
- US-A1- 2009 264 673

## Description

La présente invention concerne la préparation d'anhydride (méth)acrylique par réaction d'acide (méth)acrylique avec un anhydride autre qu'un anhydride (méth)acrylique, selon une réaction dite de transanhydrification.

Les anhydrides (méth)acryliques sont classiquement préparés par une réaction de transanhydrification, typiquement par réaction d'acide (méth)acrylique avec de l'anhydride acétique, ce par quoi il se forme de l'acide acétique et l'anhydride recherché. L'acide acétique formé est généralement éliminé par distillation au fur et à mesure de sa formation. Ce type de réaction, bien connu, est décrit par exemple dans la demande EP 1 273 565.

En marge de la préparation de l'anhydride (méth)acrylique souhaité, il se forme des sous-produits, notamment des produits de polymérisation et des produits d'addition, qui nécessitent une purification de l'anhydride (méth)acrylique formé. La teneur en ces sous-produits peut être réduite de façon connue en soi par l'addition d'inhibiteurs de polymérisation. Néanmoins, même en employant de tels inhibiteurs, il continue à se former des sous-produits. Typiquement, ces sous-produits sont éliminés par distillation, ce qui est une opération délicate, notamment compte tenu du caractère lacrymogène de l'anhydride (méth)acrylique.

Des procédés de synthèse d'anhydride (méth)acrylique de type « batch » (procédés discontinus par lots) ont notamment été décrits, par exemple dans la demande EP 0 231 689.

Alternativement, des procédés continus et semi-continus, plus intéressants, ont également été proposés, notamment dans EP 1 237 565 ou US 2009/0264673, et permettent de réduire la présence de sous-produits.

Par ailleurs, il a été envisagé l'emploi de catalyseurs pour améliorer la réaction. Dans ce cadre, pour l'essentiel, ce sont des catalyseurs hétérogènes qui ont été envisagés, notamment dans US 2002/0161260, et qui peuvent poser des difficultés en termes d'extrapolation ou de transfert de matière. Plus ponctuellement, il a été proposé des catalyseurs homogènes, comme par exemple l'acide sulfurique décrit dans DE 3510035, qui ne posent pas ce type de difficulté, mais qui, en contrepartie, présentent le plus souvent un inconvénient majeur, à savoir qu'ils impliquent en général des étapes lourdes de post-traitement pour être séparés de l'anhydride. Ainsi, ces catalyseurs ne conduisent pas systématiquement à une amélioration du rendement et présentent en outre souvent l'inconvénient de devoir être éliminés à l'issue de la réaction. Un but de la présente invention est de fournir un procédé de préparation d'anhydride (méth)acrylique évitant des étapes lourdes de post-traitement de l'anhydride, notamment des étapes lourdes d'élimination du catalyseur utilisé.

La présente invention vise aussi à fournir un procédé efficace de préparation d'anhydride (méth)acrylique, qui puisse, au besoin, être mis en oeuvre selon un mode continu, en évitant les lourdeurs et inconvénients des procédés de type « batch ».

A cet effet, la présente invention propose de mettre en oeuvre un catalyseur particulier, à savoir l'acide triflique sous forme hydratée.

Dans le cadre des travaux qui ont conduit à la présente invention, les inventeurs ont mis en évidence que l'acide triflique est un catalyseur particulièrement intéressant pour la réaction transanhydrification. Les inventeurs ont mis en évidence que l'acide triflique est un catalyseur particulièrement réactif, induisant un faible temps de séjour dans la zone de réaction et autorisant la mise en oeuvre du procédé en continu. L'acide triflique présente en outre la propriété, lorsqu'il est anhydre, d'être plus volatil que l'anhydride formé dans la réaction et qui peut de ce fait être aisément séparé et recyclé.

Les inventeurs ont plus spécifiquement mis en évidence que, de façon inattendue, lorsque l'acide triflique est utilisé en présence de traces d'eau dans la réaction de transanhydrification (typiquement de l'eau contenue à l'état de traces dans l'acide (méth)acrylique de départ), l'acide triflique est converti en tout ou partie en sa forme hydratée (en général, l'acide triflique est totalement converti en sa forme hydratée compte tenu de la faible quantité d'acide triflique employé et de la forte réactivité de cet acide).

Comme illustré dans l'exemple 2 ci-annexé, l'acide triflique sous sa forme hydratée présente un réactivité simialire à celle de la forme anhydre et reste donc intéressante pour promouvoir la réaction de transanhydrification. Cependant, contrairement à l'acide triflique sous forme anhydre, la forme hydratée de l'acide triflique s'avère moins volatile que l'anhydride (méth)acrylique formé à l'issue de la réaction et ne peut donc être séparée aussi aisément que la forme anhydre.

Néanmoins, les inventeurs ont mis au point un procédé aisé à mettre en oeuvre permettant de séparer facilement l'anhydride méthacrylique à l'issue de la réaction, ledit procédé faisant l'objet de la présente invention.

Plus précisément, il est proposé selon la présente invention un procédé de préparation d'un anhydride de formule A-C(=O)-O-(O=)C-A, où A est un groupement -CH=CH₂ ou -C(CH₃)=CH₂, ledit procédé comprenant :
a) une étape de réaction d'un anhydride B-C(=O)-O-(O=)C-B avec un acide A-COOH, A étant tel que défini ci-dessus, cette étape conduisant à la formation d'un anhydride A-C(=O)-O-(O=)C-B et d'un acide B-COOH, A et B étant tels que ledit acide B-COOH est plus volatil que ledit acide A-COOH ; et
b) une étape de réaction dudit anhydride A-C(=O)-O-(O=)C-B avec l'acide A-COOH dans des conditions telles que la quantité d'acide B-COOH est inférieure à la quantité d'acide A-COOH, conduisant à la formation de l'anhydride A-C(=O)-O-(O=)C-A,
où :
∘ lesdites étapes (a) et (b) sont mises en oeuvre en présence d'acide triflique sous forme hydratée
∘ l'anhydride A-C(=O)-O-(O=)C-A formé dans l'étape (b) est isolé du milieu réactionnel issu de l'étape (b) selon les étapes de séparation suivantes, successives ou concomitantes :
   e1) on sépare dudit milieu réactionnel, typiquement par distillation fractionnée, les composés, dits composés lourds, ayant une volatilité inférieure ou égale à celle l'anhydride A-C(=O)-O-(O=)C-A,
      ces composés lourds incluant l'anhydride A-C(=O)-O-(O=)C-A et l'acide triflique sous forme hydratée ;
   e2) on sépare l'anhydride A-C(=O)-O-(O=)C-A des composés lourds par différence de volatilité.

Dans le cadre de la présente description, le terme « (méth)acrylique » est employé dans la présente description comme synonyme de l'expression « acrylique et/ou méthacrylique ». Ainsi, lorsqu'il est fait référence à un acide (méth)acrylique, ce terme désigne l'acide acrylique CH₂=CH-COOH ou l'acide méthacrylique CH₂=C(CH₃)-COOH, ou bien encore un mélange de ces deux acides. De la même façon, si on fait référence à un anhydride (méth)acrylique, on entend désigner un anhydride acrylique CH₂=CH-C(=O)-O-(O=)C-CH=CH₂, un anhydride méthacrylique CH₂=C(CH₃)-C(=O)-O-(O=)C-C(CH₃)=CH₂, un anhydride mixte acrylique et méthacrylique CH₂=CH-C(=O)-O-(O=)C-C(CH₃)=CH₂, ou bien un mélange de ces anhydrides.

Le procédé de l'invention permet de préparer les différentes espèces répondant au terme « anhydride (méth)acrylique », à savoir de l'anhydride acrylique, de l'anhydride méthacrylique, ou un mélange des deux, en fonction du choix de l'acide A-COOH employé dans le procédé.

Selon un mode de réalisation intéressant, l'anhydride (méth)acrylique préparé selon l'invention est soit un anhydride acrylique CH₂=CH-C(=O)-O-(O=)C-CH=CH₂, soit un anhydride méthacrylique CH₂=C(CH₃)-C(=O)-O-(O=)C-C(CH₃)=CH₂, respectivement en partant d'acide acrylique ou d'acide méthacrylique à titre d'acide A-COOH. Il n'est toutefois pas exclu, selon un mode de réalisation plus particulier, de partir d'un mélange d'acides acrylique et méthacrylique.

Selon l'invention, le procédé de préparation de l'anhydride (méth)acrylique comprend une étape, notée a), de réaction d'un anhydride B-C(=O)-O-(O=)C-B avec de l'acide triflique sous forme hydratée.

Au sens de la présente invention, l'expression « acide triflique sous forme hydratée » désigne de l'acide triflique lié à de l'eau. L'acide trifique sous forme hydraté, qui se forme lorsque l'acide est mis en contact avec de l'eau se comporte comme un composé défini, distinct de l'acide triflique et s'en distingue notamment par une volatilité plus faible que celle de l'acide triflique anhydre.

Le choix d'un anhydride B-C(=O)-O-(O=)C-B tel que B-COOH est plus volatil que A-COOH, conduit à ce que chacun des produits qui peuvent se former au cours de l'étape de réaction soit plus volatil que l'anhydride (méth)acrylique A-C(=O)-O-(O=)C-A. Ce dernier, moins volatil, est alors généralement plus facile à isoler du reste des composés présents, lors d'étapes de traitement ultérieures.

Cette étape a) conduit à la formation d'un composé de type anhydride mixte A-C(=O)-O-(O=)C-B, plus volatil que l'anhydride A-C(=O)-O-(O=)C-A, et d'un acide B-COOH.

Selon l'invention, le procédé de préparation de l'anhydride (méth)acrylique comprend également une étape, notée b), de réaction dudit anhydride A-C(=O)-O-(O=)C-B avec l'acide A-COOH dans des conditions telles que la quantité d'acide B-COOH est inférieure à la quantité d'acide A-COOH.

Dans de telles conditions, l'étape b) conduit à la formation de l'anhydride A-C(=O)-O-(O=)C-A.

En effet, lorsque la quantité d'acide B-COOH est inférieure à la quantité d'acide A-COOH, l'équilibre de la réaction tend vers la formation de l'anhydride souhaité. Ainsi, l'anhydride mixte A-C(=O)-O-(O=)C-B réagit avec l'acide A-COOH, conduisant à la formation de l'anhydride A-C(=O)-O-(O=)C-A et d'acide B-COOH.

Si de telles conditions ne sont pas vérifiées, alors l'anhydride mixte ne peut pas réagir avec l'acide A-COOH pour former l'anhydride A-C(=O)-O-(O=)C-A, il peut donc se retrouver parmi les produits finaux.

Ainsi, au cours du procédé, lorsque la quantité d'acide B-COOH devient supérieure à la quantité d'acide A-COOH, la deuxième étape de réaction ne peut plus avoir lieu. On obtient alors parmi les produits finaux à la fois de l'anhydride A-C(=O)-O-(O=)C-A et de l'anhydride mixte A-C(=O)-O-(O=)C-B. Le fait que ce dernier soit plus volatil que l'anhydride A-C(=O)-O-(O=)C-A permet de le séparer aisément de celui-ci.

Dans le cadre de l'invention, les étapes de réaction a) et b) peuvent se dérouler simultanément ou de façon séquentielle.

L'ensemble des deux étapes de réaction a) et b) du procédé selon l'invention peut être désigné par « réaction de transanhydrification ».

Ainsi, l'anhydride (méth)acrylique est formé selon une réaction de transanhydrification, c'est-à-dire partant de l'acide (méth)acrylique et d'un anhydride B-C(=O)-O-(O=)C-B, différent de l'anhydride souhaité, et conduisant à l'anhydride (méth)acrylique souhaité et un acide carboxylique B-COOH, différent de l'acide initial, avec comme composé intermédiaire entre les anhydrides initial et final, l'anhydride A-C(=O)-O-(O=)C-B.

On peut associer à cette réaction de transanhydrification une zone de réaction qui délimite l'espace dans lequel se déroulent les deux étapes a) et b).

Ainsi, différents composés peuvent être présents dans la zone de réaction et en sortie de cette zone.

Plus précisément, à un instant donné, la zone de réaction comprend généralement de l'anhydride B-C(=O)-O-(O=)C-B, de l'acide A-COOH, le catalyseur, le composé mixte A-C(=O)-O-(O=)C-B, de l'acide B-COOH et de l'anhydride A-C(=O)-O-(O=)C-A. L'ensemble de ces composés peut également être présent en sortie de la zone de réaction.

En effet, comme indiqué plus haut, les réactifs B-C(=O)-O-(O=)C-B et A-COOH, ainsi que le catalyseur, réagissent dans la zone de réaction pour d'abord former l'anhydride mixte A-C(=O)-O-(O=)C-B et l'acide B-COOH. Puis, si la quantité d'acide B-COOH est inférieure à celle d'acide A-COOH, l'anhydride mixte réagit alors avec l'acide A-COOH et de l'anhydride A-C(=O)-O-(O=)C-A est formé.

Selon l'invention, l'anhydride B-C(=O)-O-(O=)C-B et l'acide A-COOH sont plus volatils que l'anhydride préparé. L'anhydride formé est ainsi moins volatil que les réactifs, ce qui facilite son isolement, même lorsqu'une certaine quantité de réactifs n'a pas réagi et se retrouve parmi les composés formés pendant la réaction.

Ainsi, l'anhydride B-C(=O)-O-(O=)C-B, l'acide A-COOH et l'acide B-COOH sont plus volatils que l'anhydride A-C(=O)-O-(O=)C-A formé. L'anhydride formé, et l'acide triflique plus lourd, sont donc les composés les moins volatils parmi les produits et réactifs (ils constituent la fraction lourde, aisée à séparer des autres produits, réactifs et intermédiaires réactionnels). La séparation de l'anhydride hors de cette fraction lourde peut être aisément effectuée selon les étapes de séparation (e1) et (e2).

Selon un premier mode de réalisation, les étapes (e1) et (e2) sont mises en oeuvre successivement, à savoir en séparant les composés lourds dans l'étape (e1) et en traitant en aval ces composés lourds dans l'étape de séparation (e2) pour extraire l'anhydride recherché. Dans ce cas, l'étape (e2) est typiquement conduite en aval de l'étape (e1), à l'aide d'une colonne à distiller ou à l'aide d'un appareil à court temps de séjour tel qu'un évaporateur à film tombant, ou bien encore un évaporateur à film raclé. L'étape (e2) peut par exemple être conduite dans un dispositif à court trajet du type du type décrit dans les brevets US 3,434,935 ou US 4,517,057.

Selon un autre mode de réalisation possible, les étapes (e1) et (e2) peuvent être conduite simultanément, typiquement au sein d'une même colonne de distillation où les composés autres que les composés lourds sont extraits en tête de colonne et les composés lourds en partie basse, en soutirant séparément :
- dans la partie la plus basse de la colonne :
   les composés les plus lourds, incluant l'acide triflique hydraté
- dans une partie plus haute :
   l'anhydride méth(acrylique).

Selon une variante possible de l'invention, compatible avec les différents modes envisagés plus haut dans la présente description, l'acide B-COOH est éliminé au cours des étapes a) et b).

Cette élimination de l'acide B-COOH peut par exemple effectuée par distillation.

Comme mentionné plus haut, au cours de l'étape b) l'anhydride mixte A-C(=O)-O-(O=)C-B peut réagir avec l'acide A-COOH pour donner l'anhydride souhaité A-C(=O)-O-(O=)C-A, si la quantité d'acide B-COOH est inférieure à celle d'acide A-COOH. Par conséquent, afin d'obtenir de telles conditions et de déplacer l'équilibre de la réaction vers la formation de l'anhydride souhaité, l'acide B-COOH formé peut être éliminé au cours des étapes a) et b), c'est-à-dire retiré de la zone de réaction.

L'élimination progressive de l'acide B-COOH permet ainsi d'obtenir des conditions permettant la formation de l'anhydride souhaité A-(C=O)-O-(C=O)-A.

Selon un mode de réalisation particulier, compatible avec les précédents, les étapes (a) et (b) du procédé sont effectuées en continu.

Dans le cadre de la présente description, on entend par « procédé effectué en continu » ou plus simplement « procédé continu » un procédé dont les différentes opérations successives s'enchaînent sans interruption et par conséquent dans lequel le produit, ici l'anhydride (méth)acrylique, est élaboré de façon ininterrompue.

Ainsi, selon ce mode, les réactifs sont introduits de façon continue et les composés susceptibles d'être obtenus sont récupérés également de façon continue. Le procédé de l'invention peut donc soit être mis en oeuvre de façon non continue, on le désigne alors par « procédé batch » ou « procédé en mode batch » (par lots), soit de façon continue, on la désigne alors par « procédé continu ».

Selon un mode de réalisation intéressant de l'invention, également compartible avec tous les modes précédents, la masse molaire de B est inférieure à la masse molaire de A. Dans ce cadre, B est de préférence un groupe méthyle ou éthyle, avantageusement un groupe méthyle.

Ainsi, la préparation de l'acide (méth)acrylique paut typiquement être effectuée par réaction d'un acide (méth)acrylique avec l'anhydride acétique. Par exemple, l'acide méthacrylique peut être préparé par réaction d'acide méthacrylique avec de l'anhydride acétique.

La température de mise en oeuvre est de préférence choisie de façon à obtenir cinétique suffisamment rapide, tout en évitant une dégradation trop importante des composés. Pour obtenir une cinétique suffisamment rapide, la température de mise e oeuvre des étapes (a) et (b) est de préférence supérieure à 60°C, par exemple d'au moins 70°C, voire d'au moins 90°C. Pour éviter que la température ne dégrade les composés, il est toutefois préférable de conduire les étapes (a) et (b) à une température restant inférieure ou égale à 120°C, avantageusement inférieure ou égale à 110°C, par exemple inférieure ou égale à à 100°C.

Selon un mode de réalisation intéressant, les étapes de réaction a) et b) sont conduites à une pression de 0,01 bar à 3 bar, avantageusement de 0,5 bar à 1,5 bar, de préférence à pression atmosphérique.Au sens de la présente invention, on entend par « pression atmosphérique » la pression ambiante qui règne dans les conditions du procédé, égale à 1 bar ou avoisinant 1 bar.

Le procédé peut être mis en œuvre quelle que soit la pression, mais il est particulièrement avantageux de travailler à pression atmosphérique, étant donné que cela permet de s'affranchir de tout contrôle de la pression.

Avantageusement, le ratio molaire entre l'acide A-COOH et l'anhydride B-C(=O)-O-(O=)C-B est de 0,5 à 5, avantageusement de 1,5 à 3. De préférence ce ratio molaire A-COOH : B-C(=O)-O-(O=)C-B reste inférieur à 2,5 et plus préférentiellement inférieur à 2.

Avantageusement, le ratio entre la masse de catalyseur acide et la masse totale des réactifs B-C(=O)-O-(O=)C-B et A-COOH est de 5 ppm à 1%, avantageusement de 20 ppm à 100 ppm.

Selon un mode de réalisation, le procédé comprend une étape d'extraction du catalyseur acide et de l'acide B-COOH formé, pour séparer le catalyseur acide et l'acide B-COOH, notamment par distillation. Cette étape d'extraction se déroule alors après les étapes a) et b).

A l'issue de la réaction de transanhydrification, les principaux composés présents sont le catalyseur acide, l'acide B-COOH et l'anhydride A-C(=O)-O-(O=)C-A. Ainsi, le catalyseur acide et l'acide B-COOH formé sont extraits notamment par distillation afin d'isoler l'anhydride A-C(=O)-O-(O=)C-A et de recycler le cas échéant le catalyseur pour qu'il soit réutilisable. Dans ce cas, le catalyseur peut être réutilisé dans l'étape de réaction susmentionnée.

On peut ainsi définir une zone d'extraction délimitant l'espace dans lequel se déroule l'étape d'extraction. Les zones de réaction et d'extraction peuvent être distinctes, confondues ou se chevaucher, comme décrit plus loin.

Avantageusement, après l'étape d'extraction, le catalyseur acide est récupéré et recyclé pour être utilisé dans l'étape de réaction de l'acide A-COOH avec l'anhydride B-C(=O)-O-(O=)C-B.

Divers traitements permettant de récupérer le catalyseur dans le mélange catalyseur/acide B-COOH extrait après l'étape de réaction sont envisageables. De préférence, la récupération du catalyseur est effectuée par distillation.

Comme indiqué plus haut, lorsque le catalyseur acide est moins volatil que l'acide B-COOH, la séparation entre le catalyseur et l'acide B-COOH est plus facile. Cette séparation est typiquement effectuée par distillation.

Les étapes de réaction sont généralement effectuées dans un réacteur.

Ce réacteur définit alors la zone de réaction. Il s'agit généralement d'un réacteur piston ou d'un réacteur continu agité (typiquement de type parfaitement agité), ou encore d'une cascade de réacteurs continus agités.

Avantageusement, l'étape d'extraction est effectuée dans une colonne de distillation.

Cette colonne définit alors la zone d'extraction.

La colonne possède par exemple de 10 à 30 plateaux théoriques, par exemple 25 à 30 plateaux théoriques.

Du fait des différences de volatilité entre les différents composés obtenus, les produits les plus lourds, à savoir l'acide triflique sous forme hydratée et l'anhydride (méth)acrylique, sont récupéré en pied de colonne, tandis que l'acide B-COOH sont récupérés en tête de colonne.

Selon un mode de réalisation, les étapes de réaction a) et b) sont effectuées dans un réacteur et l'étape d'extraction est effectuée dans une ou plusieurs colonnes de distillation successives, distinctes du réacteur.

Ainsi, les divers réactifs, à savoir B-C(=O)-O-(O=)C-B, A-COOH et le catalyseur, réagissent au sein du réacteur, dans la zone de réaction, pour former l'anhydride A-C(=O)-O-(O=)C-B, l'acide B-COOH, et l'anhydride (méth)acrylique souhaité de façon quantitative si les conditions requises sont vérifiées. Le réacteur étant relié à la colonne de distillation, au fur et à mesure que se déroulent les étapes a) et b) dans le réacteur, les différents produits (A-C(=O)-O-(O=)C-A, B-COOH et éventuellement A-C(=O)-O-(O=)C-B) et/ou les réactifs migrent vers la colonne de distillation, définissant la zone d'extraction, qui permet de séparer l'anhydride formé des autres composés présents.

Le réacteur et la colonne sont alors des entités distinctes.

Ainsi, même si le réacteur est distinct de la colonne, lorsque les deux entités sont reliées, la réaction de transanhydrification peut également se dérouler dans la colonne. Les zones de réaction et d'extraction sont donc alors initialement distinctes, mais peuvent se chevaucher.

L'avantage de ce mode réside dans la possibilité de choisir les paramètres de réaction au sein du réacteur (tels que la température, la pression ou les quantités de réactifs) indépendamment des conditions de séparation dans la colonne.

Selon un mode de réalisation intéressant, un ou plusieurs inhibiteurs de polymérisation peuvent être introduits au cours du procédé afin de limiter la formation de sous-produits tels que des polymères à base d'acide (méth)acrylique et/ou d'anhydride (méth)acrylique.

Ils peuvent alors être introduits avec l'acide A-COOH dans la zone de réaction et/ou dans la zone d'extraction.

Avantageusement, ces inhibiteurs sont introduits dans la zone d'extraction. En effet, ils sont particulièrement efficaces dans la zone d'extraction et permettent notamment d'éviter les réactions de condensation susceptibles de s'y produire.

De préférence, lorsque la zone d'extraction consiste en une colonne de distillation, les inhibiteurs sont introduits en tête de colonne.

Ces inhibiteurs doivent, le cas échéant, être actifs vis-à-vis de la polymérisation tout en étant inertes à l'égard des anhydrides et de l'acide (méth)acrylique. Ils peuvent notamment être choisis parmi l'hydroquinone, l'hydroquinone monométhyl éther, le topanol A, la phénothiazine et l'hydroxytétraméthylpipéridinoxyl (hydroxy-TEMPO).

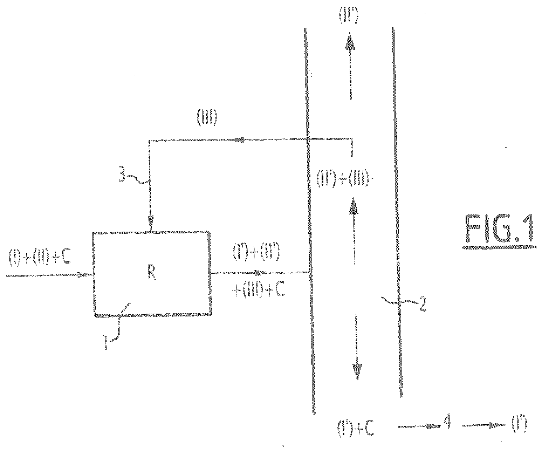

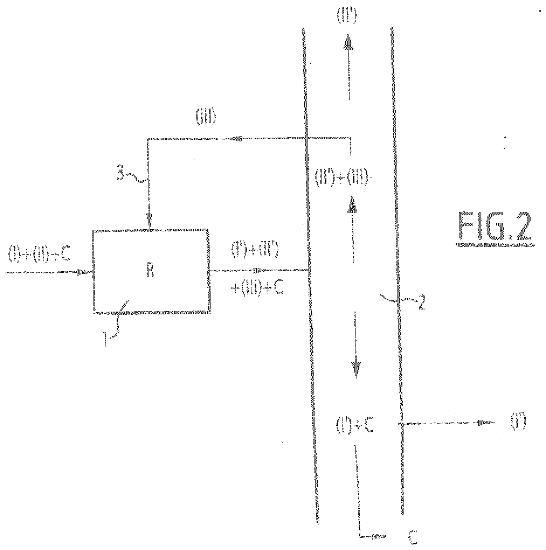

L'invention est encore davantage illustrée par de la description qui suit, donnée uniquement à titre d'exemple et faite en référence aux dessins ci-annexés, où :
**la** **Figure 1** est une vue schématique en coupe selon un plan vertical médian d'un premier type de dispositif adapté à la mise en oeuvre du procédé de l'invention selon un mode continu avec une mise en oeuvre de l'étape (e2) en aval de l'étape (e1), du type employé dans l'exemple 1 ci-après ;
**la** **Figure 2** est une vue schématique en coupe selon un plan vertical médian d'un second type de dispositif adapté à la mise en oeuvre du procédé de l'invention selon un mode continu, pour une mise en oeuvre concomitante des étapes de séparation (e1) et (e2).

Les dispositifs représentés sur les Figure 1 et 2 sont des dispositifs similaires qui se distinguent uniquement par les moyens employés pour séparer l'anhydride formé. Ces dispositifs comportent un réacteur 1, typiquement un réacteur de type parfaitement agité, ou de type piston, en connection fluide avec une zone intermédiaire d'une colonne de distillation 2, dont la partie haute est de préférence également en connection fluide avec le réacteur 1, *via* une boucle de réinjection 3. La boucle de réinjection est optionnelle mais fortement recommandée en pratique, cette boucle permettant une amélioration du rendement.

Le procédé de l'invention est typiquement mis en oeuvre dans les conditions suivantes dans les dispositifs des Figures 1 et 2 :
De l'anhydride B-C(=O)-O-(O=)C-B, par exemple de l'anhydride acétique, désigné par la référence générale (I) sur les figures, de l'acide (méth)acylique A-COOH, désigné par la référence générale (II), et de l'acide triflique, noté C sont injectés en continu dans le réacteur 1. L'acide triflique est employé sous forme hydraté, ce qui peut être obtenu par exemple en employant de l'acide (méth)acrylique comprenant des traces d'eau. On peut introduire en outre dans le réacteur d'autres composés, comme par exemple des inhibiteurs de polymérisation.

L'étape a) se déroule alors dans une zone de réaction du réacteur, notée R, et conduit à la formation d'anhydride mixte A-C(=O)-O-(O=)C-B, noté (III), et d'acide B-COOH, noté (II'), qui est typiquement de l'acide acétique.

Afin que l'étape b) puisse avoir lieu, l'acide (II') est éliminé au fur et à mesure de sa formation, pour que sa quantité dans la zone R soit inférieure à celle de l'acide (II). L'étape b) se déroule alors dans la zone R et conduit à la formation de l'anhydride (I').

Pour ce faire, à la sortie du réacteur 1, les réactifs (I), (II) et C, le composé intermédiaire (III) et les produits (I') et (II') sont acheminés vers la colonne de distillation 2. Les produits les plus lourds, incluant L'anhydride (I') et l'acide triflique sous forme hydratée (et les inhibiteurs de polymérisation, le cas échéant) migrent vers le bas de la colonne alors que les composés plus légers sont entraînés vers la tête. L'acide (II'), qui est le composé le plus léger, migre en tête de colonne, où il est extrait en tout ou partie (généralement, on récupère la quasi-totalité de l'acide (II') en tête de colonne, et le reste des composés légers, l'anhydride mixte (III), des réactifs (I) et (II) n'ayant pas réagi, et éventuellement de l'acide (II') en faible quantité, est extrait plus bas que la tête de la colonne, pour être réinjecté dans le réacteur 1 au moyen de la boucle 3.

L'anhydride (I') est récupéré à partir des composés lourds qui migrent vers le bas de la colonne selon deux modes possibles :
- la Figure 1 illustre un mode en deux étapes : les composés lourds sont tous récupérés en pied de colonne (étape e1) puis sont séparés dans un dispositif 4 où on effectue une séparation de l'anhydride (I'), qui est le plus léger des composés lourds (étape e2). Le dispositif 4 peut typiquement être une colonnne de distillation (où l'anhydride (I') est récupéré en tête de colonne), ou bien tout autre dispositif apte à séparer le composé le plus volatil d'un mélange, tel qu'un évaporateur à film tombant, ou bien encore un évaporateur à film raclé
- la Figure 2 illustre un mode en une seule étape : la séparation des composés lourds est effectuée dans la partie basse de la colonne, en récupérant les composés les plus lourds en pied de colonne et en extrayant l'anhydride (I') plus haut que le pied de la colonne.

A noter que les figures 1 et 2 représentent deux modes de réalisation particuliers et non limitatifs de l'invention.

D'autres variantes peuvent être mises en oeuvre. Par exemple, la séparation effectuée au sein d'une unique colonne de distillation dans les dispositifs des Figures 1 et 2 peut, alternativement être réalisé dans des colonnes successives.

Ainsi, par exemple, au lieu de séparer les composés les plus volatils dans la colonne 2, il peut être envisagé de mettre la partie haute de la colonne 2 en connexion fluide avec une seconde colonne (non représentée sur les figures) et de récupérer l'acide (I') en tête de cette seconde colonne et les autres composés en pied de la seconde colonne, pour renvoyer ces autres composés vers le réacteur 1.

### EXEMPLES

### Exemple 1 :

### Préparation d'anhydride méthacrylique selon l'invention

Dans cet exemple, on a employé un dispositif de type illustré sur la figure 1, sans boucle de recyclage 3, où le réacteur 1 est un réacteur agité d'un volume de 5L (volume suffisant pour atteindre l'équilibre à la sortie à 80-90°C à la pression atmosphérique), et où la colonne de distillation 2 est une colonne de 3 cm de diamètre comportant 30 plateaux et au sein de laquelle on a instauré un vide de 2000 Pa (20 mbar) assuré par une pompe à palette et le dispositif 4 est un évaporateur à film raclé

Dans cet exemple on a introduit les réactifs avec un flux entrant de 630 g/h d'acide méthacrylique et un flux entrant de 370 g/h d'anhydride acétique, ainsi que des inhibiteurs de polymérisation (phénothiazine). La réaction a été conduite en continu dans un réacteur parfaitement agité de volume suffisant pour atteindre l'équilibre à la sortie à 80-90°C à la pression atmosphérique.

Au niveau de la colonne de distillation, le flux sortant en pied (anhydride brut contenant 93 à 95% en masse l'anhydride méthacrylique et l'acide triflique hydraté) était de 200 g/h.

Le flux d'anhydride brut sortant en pied de la colonne a été purifié sur un évaporateur à film raclé, pour obtenir l'anhydride purifié.

Des résultats similaires, mais avec un rendement bien plus élevé sont obtenus lorsqu'on met en oeuvre une boucle de recirculation du type de la boucle 3 de la Figure 1 ci-attachée.

### Exemple 2 :

### réactivité de l'acide triflique sous forme anhydre et sous forme hydratée

Cet exemple illustre les réactivités similaires de l'acide triflique sous forme anhydre et sous forme hydratée.

Dans un réacteur en verre de volume 1 litre agité mécaniquement et à double enveloppe, maintenu à 80°C à la pression atmosphérique, on a introduit: 150 g d'anhydride acétique, 250 g d'acide méthacrylique, 0.8 g de phénothiazine et, selon l'essai :
- essai 1 : 25 ppm d'acide triflique anhydre
- essai 2 : 25 ppm d'acide triflique sous forme hydratée

On a maintenu le réacteur sous agitation à 80°C sous pression atmosphérique et on a effectué des prélèvements au cours du temps, pour déterminer le temps nécessaire pour atteindre l'équilibre.

A titre comparatif : on a réalisé un témoin en effectuant l'expérience dans les mêmes conditions que les essais 1 et 2, mais sans aucun ajout d'acide triflique.

Les résultats obtenus sont reportés dans le tableau ci-dessous, qui montre des résultats similaires pour les deux formes de l'acide triflique :

| | **temps pour atteindre l'équilibre (en minutes)** |
|---|---|
| ***Témoin :*** | 140 |
| pas d'acide triflique ajouté | |
| ***Essai* 1 :** | 27 |
| Ajout d'acide triflique anhydre (25 ppm) | |
| ***Essai* 2 :** | 25 |
| Ajout d'acide triflique hydraté (25 ppm) | |

## Revendications

1. Procédé de préparation d'un anhydride de formule A-C(=O)-O-(O=)C-A, où A est un groupement -CH=CH₂ ou -C(CH₃)=CH₂, ledit procédé comprenant :
a) une étape de réaction d'un anhydride B-C(=O)-O-(O=)C-B avec un acide A-COOH, A étant tel que défini ci-dessus, cette étape conduisant à la formation d'un anhydride A-C(=O)-O-(O=)C-B et d'un acide B-COOH, A et B étant tels que ledit acide B-COOH est plus volatil que ledit acide A-COOH ; et
b) une étape de réaction dudit anhydride A-C(=O)-O-(O=)C-B avec l'acide A-COOH dans des conditions telles que la quantité d'acide B-COOH est inférieure à la quantité d'acide A-COOH, conduisant à la formation de l'anhydride A-C(=O)-O-(O=)C-A,
où :
o lesdites étapes (a) et (b) sont mises en oeuvre en présence d'acide triflique sous forme hydratée
o l'anhydride A-C(=O)-O-(O=)C-A formé dans l'étape (b) est isolé du milieu réactionnel issu de l'étape (b) selon les étapes de séparation suivantes, successives ou concomitantes :
e1) on sépare dudit milieu réactionnel, typiquement par distillation fractionnée, les composés, dits composés lourds, ayant une volatilité inférieure ou égale à celle l'anhydride A-C(=O)-O-(O=)C-A,
ces composés lourds incluant l'anhydride A-C(=O)-O-(O=)C-A et l'acide triflique sous forme hydratée ;
e2) on sépare l'anhydride A-C(=O)-O-(O=)C-A des composés lourds, par différence de volatilité.

2. Procédé selon la revendication 1, dans lequel l'acide B-COOH est éliminé au cours des étapes de réaction a) et b).

3. Procédé selon la revendication 1 ou 2, effectué en continu.

4. Procédé selon l'une quelconque des revendications 1 à 3, dans lequel la masse molaire de B est inférieure à la masse molaire de A.

5. Procédé selon l'une quelconque des revendications 1 à 4, dans lequel B est un groupe méthyle ou éthyle, de préférence un groupe méthyle.

6. Procédé selon l'une quelconque des revendications 1 à 5, dans lequel les étapes de réaction a) et b) s'effectuent à une température de 60°C à 120°C, de préférence de 70°C à 110°C, ou de 90°C à 100°C.

7. Procédé selon l'une quelconque des revendications 1 à 6, dans lequel les étapes de réaction a) et b) s'effectuent à une pression de 0,01 bar à 3 bar, de préférence de 0,5 bar à 1,5 bar, ou à pression atmosphérique.

8. Procédé selon l'une quelconque des revendications 1 à 7, comprenant une étape d'extraction du catalyseur acide et de l'acide B-COOH formé, pour séparer le catalyseur acide et l'acide B-COOH, notamment par distillation.

9. Procédé selon la revendication 8, dans lequel les étapes de réaction sont effectuées dans un réacteur et l'étape d'extraction est effectuée dans une ou plusieurs colonnes de distillation successives, distinctes du réacteur.

10. Procédé selon la revendication 10, dans lequel les étapes de réaction et l'étape d'extraction sont effectuées dans une ou plusieurs colonnes de distillation successives.

## Patentansprüche

1. Verfahren zur Herstellung eines Anhydrids der Formel A-C(=O)-O-(O=)C-A, worin A eine Gruppe -CH=CH₂ oder -C(CH₃)=CH₂ ist, wobei das Verfahren Folgendes umfasst:
a) einen Schritt der Umsetzung eines Anhydrids B-C(=0)-0-(0=)C-B mit einer Säure A-COOH, wobei A wie vorstehend definiert ist, wobei dieser Schritt zur Herstellung eines Anhydrids A-C(=O)-O-(O=)C-B und einer Säure B-COOH führt, wobei A und B derart sind, dass die Säure B-COOH flüchtiger ist als die Säure A-COOH; und
b) einen Schritt der Umsetzung des Anhydrids A-C(=O)-O-(O=)C-B mit der Säure A-COOH unter solchen Bedingungen, dass die Menge an Säure B-COOH kleiner ist als die Menge an Säure A-COOH, was zur Herstellung des Anhydrids A-C(=O)-O-(O=)C-A führt,
wobei:
o die Schritte (a) und (b) in Gegenwart von Trifluormethansulfonsäure in hydratisierter Form durchgeführt werden
o das in Schritt (b) gebildete Anhydrid A-C(=0)-0-(0=)C-A aus dem Reaktionsmedium nach Schritt (b) gemäß den folgenden, aufeinanderfolgenden oder gleichzeitigen, Trennungsschritten isoliert wird:
e1) aus dem Reaktionsmedium werden, üblicherweise durch fraktionierte Destillation, die so genannten schweren Verbindungen mit einer Flüchtigkeit, die kleiner als oder gleich derjenigen des Anhydrids A-C(=O)-O-(O=)C-A ist, abgetrennt,
wobei diese schweren Verbindungen das Anhydrid A-C(=O)-O-(O=)C-A und Trifluormethansulfonsäure in hydratisierter Form einschließen;
e2) das Anhydrid A-C(=O)-O-(O=)C-A wird anhand des Flüchtigkeitsunterschieds von den schweren Verbindungen abgetrennt.

2. Verfahren nach Anspruch 1, wobei die Säure B-COOH während der Umsetzungsschritte a) und b) eliminiert wird.

3. Verfahren nach Anspruch 1 oder 2, das kontinuierlich durchgeführt wird.

4. Verfahren nach einem der Ansprüche 1 bis 3, wobei die Molmasse von B kleiner als die Molmasse von A ist.

5. Verfahren nach einem der Ansprüche 1 bis 4, wobei B eine Methyl- oder Ethylgruppe, vorzugsweise eine Methylgruppe, ist.

6. Verfahren nach einem der Ansprüche 1 bis 5, wobei die Umsetzungsschritte a) und b) bei einer Temperatur von 60°C bis 120°C, vorzugsweise von 70°C bis 110°C oder von 90°C bis 100°C, durchgeführt werden.

7. Verfahren nach einem der Ansprüche 1 bis 6, wobei die Umsetzungsschritte a) und b) bei einem Druck von 0,01 bar bis 3 bar, vorzugsweise von 0,5 bar bis 1,5 bar oder bei Atmosphärendruck, durchgeführt werden.

8. Verfahren nach einem der Ansprüche 1 bis 7, umfassend einen Schritt der Extraktion des Säurekatalysators und der gebildeten Säure B-COOH, um den Säurekatalysator und die Säure B-COOH, insbesondere durch Destillation, zu trennen.

9. Verfahren nach Anspruch 8, wobei die Umsetzungsschritte in einem Reaktor durchgeführt werden und der Extraktionsschritt in einer oder mehreren aufeinanderfolgenden, vom Reaktor getrennten Destillationskolonnen durchgeführt wird.

10. Verfahren nach Anspruch 10, wobei die Umsetzungsschritte und der Extraktionsschritt in einer oder mehreren aufeinanderfolgenden Destillationskolonnen durchgeführt werden.

## Claims

1. Process for preparing an anhydride of formula A-C(=O)-O-(O=)C-A, wherein A is a -CH=CH₂ or -C(CH₃)=CH₂ group, said process comprising:
a) a step of reacting an anhydride B-C(=O)-O-(O=)C-B with an acid A-COOH, A being as defined above, this step resulting in the formation of an anhydride A-C(=O)-O-(O=)C-B and of an acid B-COOH, A and B being such that said acid B-COOH is more volatile than said acid A-COOH; and
b) a step of reacting said anhydride A-C(=O)-O-(O=)C-B with the acid A-COOH under conditions such that the amount of acid B-COOH is less than the amount of acid A-COOH, resulting in the formation of the anhydride A-C(=O)-O-(O=)C-A,
wherein:
∘ said steps (a) and (b) are carried out in the presence of triflic acid in hydrated form
∘ the anhydride A-C(=O)-O-(O=)C-A formed in step (b) is isolated from the reaction medium resulting from step (b) according to the following successive or concomitant separation steps:
e1) the compounds, termed heavy compounds, having a volatility of less than or equal to that of the anhydride A-C(=O)-O-(O=)C-A are separated from said reaction medium, typically by fractional distillation,
these heavy compounds including the anhydride A-C(=O)-O-(O=)C-A and triflic acid in hydrated form;
e2) the anhydride A-C(=O)-O-(O=)C-A is separated from the heavy compounds, by virtue of difference in volatility.

2. Process according to Claim 1, in which the acid B-COOH is eliminated during reaction steps a) and b) .

3. Process according to Claim 1 or 2, carried out continuously.

4. Process according to one of Claims 1 to 3, in which the molar mass of B is less than the molar mass of A.

5. Process according to one of Claims 1 to 4, in which B is a methyl or ethyl group, preferably a methyl group.

6. Process according to one of Claims 1 to 5, in which reaction steps a) and b) are carried out at a temperature of from 60°C to 120°C, preferably from 70°C to 110°C, or from 90°C to 100°C.

7. Process according to one of Claims 1 to 6, in which reaction steps a) and b) are carried out at a pressure of from 0.01 bar to 3 bar, preferably from 0.5 bar to 1.5 bar, or at atmospheric pressure.

8. Process according to one of Claims 1 to 7, comprising a step of extracting the acid catalyst and the acid B-COOH formed, in order to separate the acid catalyst and the acid B-COOH, in particular by distillation.

9. Process according to Claim 8, in which the reaction steps are carried out in a reactor and the extraction step is carried out in one or more successive distillation columns, distinct from the reactor.

10. Process according to Claim 10, in which the reaction steps and the extraction step are carried out in one or more successive distillation columns.
